Europäisches Patentamt

European Patent Office

Office européen des brevets

(19)

(11) Veröffentlichungsnummer: **0 229 773**
**B1**

(12) **EUROPÄISCHE PATENTSCHRIFT**

(45) Veröffentlichungstag der Patentschrift: **16.08.90**

(51) Int. Cl.⁵: **G 01 N 21/90**

(21) Anmeldenummer: **86903359.7**

(22) Anmeldetag: **31.05.86**

(86) Internationale Anmeldenummer:
**PCT/EP86/00327**

(87) Internationale Veröffentlichungsnummer:
**WO 87/00283 15.01.87 Gazette 87/01**

(54) **VERFAHREN ZUR OPTO-ELEKTRONISCHEN INSPEKTION VON FLASCHEN.**

(30) Priorität: **04.07.85 DE 3523975**

(43) Veröffentlichungstag der Anmeldung:
**29.07.87 Patentblatt 87/31**

(45) Bekanntmachung des Hinweises auf die
Patenterteilung:
**16.08.90 Patentblatt 90/33**

(84) Benannte Vertragsstaaten:
**AT BE CH DE FR GB IT LI LU NL SE**

(56) Entgegenhaltungen:
**DE-A-2 516 138**
**DE-A-3 422 870**
**US-A-3 069 553**
**US-A-4 492 476**

**Patent Abstracts of Japan, Band 9, Nr. 90,**
**(P-350)(1813) 19. April 1985 & JP-A-59-217 141**

(73) Patentinhaber: **KRONES AG Hermann Kronseder**
**Maschinenfabrik**
**Böhmerwaldstrasse 5 Postfach 1230**
**D-8402 Neutraubling (DE)**

(72) Erfinder: **DASSLER, Hans-Ulrich**
**Mittenheimerstr. 6**
**D-8042 Oberschleissheim (DE)**
Erfinder: **HAAS, Rüdiger**
**Weidenstr. 9**
**D-8011 Faistenhaar (DE)**
Erfinder: **LÖFFLER, Gerhard**
**Karl-Theodor-Str. 93**
**D-8000 München 40 (DE)**
Erfinder: **LIEBERS, Lutz**
**Eckenhagenerstr. 4**
**D-5275 Bergneustadt (DE)**

(74) Vertreter: **Konle, Tilmar, Dipl.-Ing.**
**Benderstrasse 23 a**
**D-8000 München 60 (DE)**

Courier Press, Leamington Spa, England.

**Beschreibung**

Die Erfindung bezieht sich auf ein Verfahren gemäß dem Oberbegriff des Patentanspruchs. Ein derartiges Verfahren ist aus der US—A—4 492 476 bekannt.

Zur opto-elektronischen Inspektion der Seitenwand von gereinigten Glasflaschen auf verbliebene Verunreinigungen ist es bekannt (DE—C—32 28 464), jede Flasche auf einer Kreisbahn durch den Strahlengang zwischen einer diffus strahlenden Lichtquelle und einem elektronischen Bildwandler zu bewegen und gleichzeitig—zwecks Abwicklung der gesamten Flaschen-Seitenwand—die Flasche um 360° bezüglich ihrer Längsachse zu drehen. Der aus einer Fotodiodenkamera und einem Scanner (Drehspiegel) gebildete Bildwandler wird der Vorschubbewegung der Flasche synchron nachgeführt und dabei konstant auf die Längsachse der betreffenden Flasche ausgerichtet. Jedes in einer definierten Drehwinkelstellung der Flasche erzeugte Abtastsignal des Bildwandlers repräsentiert eine Folge übereinanderliegender Bildpunkte (Lichtlinie), welche eine Abbildung zweier radial gegenüberliegender Teilflächen der Seitenwand der Flasche darstellen. Eine Verunreinigung auf der Seitenwand verursacht einen Intensitätseinbruch des Ansonsten praktisch konstanten Abtastsignals an derjenigen Stelle im Signal, welche von den im Strahlengang durch die Verunreinigung liegenden Fotodioden der Diodenzeile erzeugt wird. Um eine Verunreinigung festzustellen, braucht lediglich der Intensitätspegel jedes Abtastsignals mit einem Minimalpegel verglichen zu werden, wobei eine Unterschreitung des Minimalpegels einen Intensitätseinbruch und damit eine Verunreinigung identifiziert. Anstelle einer Diodenzeile und eines Scanners, welcher die sich auf der Kreisbahn bewegende Flasche verfolgt, läßt sich neuerdings auch ein Diodenarray verwenden, welche aus vielen nebeneinanderliegenden Fotodidodenzeilen besteht und dessen Breite des gesamten Verfolgungsbereich abdeckt.

Mit dem bekannten Seitenwandinspektor läßt sich indessen der Mündungsbereich einer Glasflasche nicht kontrollieren. Zu diesen Mündungsbereich zählt die Ringfläche um die Flaschenmündung, welche als Dichtfläche sowohl für Kronkorken- als auch Schraubkappen-Verschlüsse dient. Bei Flaschen mit Gewindeverschluß, z.B. Mineralwasserflaschen, zählt zum Mündungsbereich ferner das Flaschengewinde. Infolge größerer Glasausbrüche in der ringförmigen Dichtfläche oder im Flaschengewinde wird der ordnungsgemäße Verschluß beeinträchtigt, so daß es wichtig ist, Flaschen mit derartigen Glasausbrüchen vor der Abfüllung auszusondern.

Bei einer bekannten Mündungskontrollvorrichtung nach der US—A—4 492 476 wird ein Fotodiodenarray so positioniert, daß es bei diffuser Beleuchtung der sich drehenden Flasche senkrecht zur Flaschenlängsachse den Mündungsbereich schräg von oben abtastet. Die spalten- und zeilenweise ausgelesenen Helligkeitswerte von aufeinanderfolgenden Bildsequenzen werden hinsichtlich einander zugeordneter Matrix-Bildpunkte untereinander sowie mit gespeicherten Referenzwerten verglichen, um Helligkeitsänderungen als Fehler zu erkennen. Die in ihrer Bildverarbeitung äußerst aufwendige Mündungskontrollvorrrichtung ist indessen für Glasflaschen mit flaschengewinde nicht geeignet, da sich in zwei aufeinanderfolgenden Matrix-Bildern die Gewindegänge infolge der Flaschendrehung verschoben haben, was bei dem bidpunktweisen Vergleich als Helligkeitsvariation und damit als Fehler mißgedeutet würde.

Bei einer bekannten Kontrollvorrichtung für Flaschengewinde nach der JP—A—59-217141 wird die senkrecht zur Flaschenlängsachse beleuchtet Flasche in ihrem Mündungsbereich mit einer ebenfalls senkrecht zur Flaschenlängsachse positionierten Kamera sequentiell abfotografiert. Das konvex-konkav variierende Bildmuster jedes Flaschengewindebildes wird mit einem zuvor gespeicherten Soll-Bildmuster hinsichtlich Abweichungen verglichen. Mit dieser Kontrollvorrichtung läßt sich allenfalls das Flaschengewinde, nicht aber die Dichtfläche überwachen. Ferner ist eine Kontrolle jeweils nur für einen speziellen Flaschentyp möglich, der zudem von der genormten und im Bildspeicher festgehaltenen Flaschen- und Gewindeform nur ganz geringfügig abweichen darf, um Fehlmeldungen zu vermeiden.

Die Aufgabe der Erfindung besteht demgegenüber darin, ein Verfahren der eingangs erwähnten Art dahingehend zu verbessern, daß es für Glasflaschen mit Flaschengewinde eine Kontrolle sowohl der Dichtfläche als auch des Flaschengewindes auf Glasausbrüche mit einer hohen Fehlererkennungszuverlässigkeit ermöglicht.

Diese Aufgabe wird erfindungsgemäß durch die kennzeichnenden Merkmale des Patentanspruchs gelöst.

Die Erfindung wird nachstehend anhand eines Ausführungsbeispiels in den Zeichnungen näher erläutert. Es zeigt:

Fig. 1 eine schematische Ansicht des Meßstrahlengangs durch eine Glasflasche bei dem erfindungsgemäßen Verfahren, und

Fig. 2 den ortsabhängigen Verlauf eines erfindungsgemäß erzeugten und ausgewerteten elektrischen Abtastsignals.

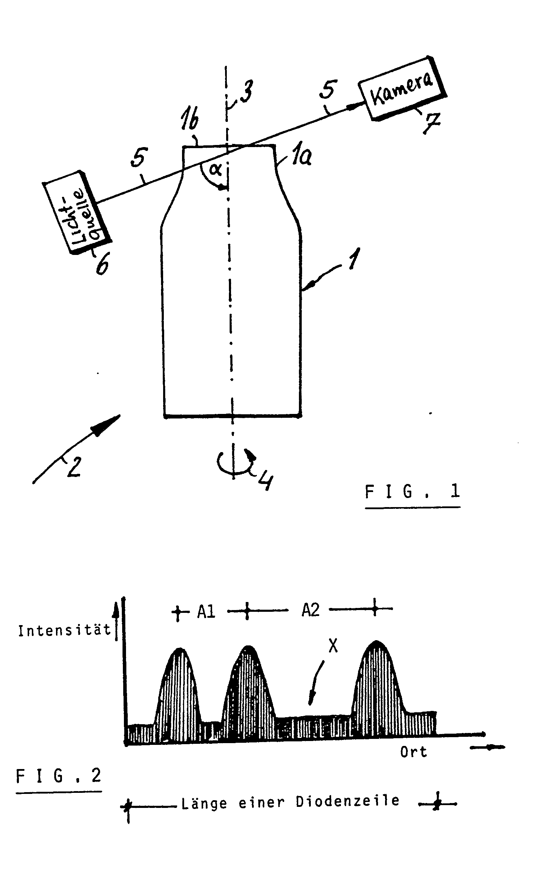

Figur 1 zeigt schematisch die Ansicht einer Glasflasche 1, welche am Mündungsbereich ein Flaschengewinde 1a sowie eine ringförmige Dichtfläche 1b aufweist. Die Flasche 1 wird längs einer mit Pfeil 2 angedeuteten Kreisbahn bewegt und gleichzeitig um ihre Längsachse 3 gedreht, was durch den Pfeil 4 angedeutet werden soll. Bei ihrem Weg längs der Kreisbahn 2 gelangt die Flasche 1 mit ihrem Mündungsbereich 1a/1b durch den Meßstrahl 5 zwischen einer hoch diffus strahlenden Lichtquelle 6 und einer Fotodiodenkamera 7. Die Kamera 7 enthält entweder eine Fotodiodenzeile und einen der Flaschenbewegung synchron nachgeführten Scanner oder ein den gesamten Flaschenverfolgungsbereich abdeckendes Fotodiodenarray.

Wesentlich ist, daß der von der Kamera 7 abgetastete Strahl 5 einerseits durch die Längsachse 3 der Flasche 1 verläuft und andererseits nicht unter einem rechten Winkel, sondern unter einem schiefen Winkel α bezüglich der Flaschenlängsachse 3 orientiert ist. Infolge dieser schiefwinkligen Durchleuchtung der Flasche 1 durchquert der Strahl 5 sowohl das Flaschengewinde 1a als auch die ringförmige Dichtfläche 1b. Die Lichtquelle 6 wird so nahe wie möglich an der Flasche 1 positiniert, um eine hohe Diffusionwirkung zu erzielen.

Das von der Kamera 7 bei jeder Abtastung, d.h. in jeder definierten Drehwinkelstellung der Flasche 1 erzeugte elektrische Signal ist in Fig. 2 schematisch dargestellt. Es besteht aus einer der Anzahl von Fotodioden je Diodenzeile entsprechenden Anzahl von Lichtintensitätswerten, wobei die Signalform entsteht, wenn die Fotodioden nacheinander ausgelesen werden. Das dargestellt Signal ist somit ortsabhängig, wobei vorausgesetzt ist, daß die Diodenzeile parallel zur Flaschenlängsachse 3 orientiert ist.

Wesentlich ist, daß die Hüllkurve des in Fig. 2 dargestellten Signals eine ausgeprägte Intensitätsmodulation aufweist. Charakteristisch dabei ist, daß an der Stelle eines Glasausbruchs im Bereich des Gewindes 1a der Flasche 1 ein Intensitätsmaximum fehlt. Eine solche Fehlerstelle ist im Signal gemäß Fig. 2 bei "X" angedeutet. Um das charakteristische Fehlen eines Maximums im Signal nach Fig. 2 und damit das Vorhandensein eines Glasausbruchs festzustellen, wird der Abstand "A" zweier benachbarter Intensitätsmaxima erfaßt und mit einem Referenzwert verglichen. Im Falle der Fig. 2 liegt der Abstand A1 unterhalb diese Referenzwertes und der Abstand A2 oberhalb dieses Referenzwertes. Das Überschreiten des Referenzwertes durch A2 identifiziert den Glasausbruch an der Stelle "X".

Es hat sich gezeigt, daß die Feststellung von Glasausbrüchen im Mündungsberich 1a/1b nach dem vorstehend erläuterten erfindungsgemäßen Verfahren eine äußerst genaue Erkennung von Glasausbrüchen, insbesondere auch von relativ kleinen Glasausbrüchen sowohl im Flaschengewinde 1a als auch in der Dichtfläche 1b ermöglicht und deshalb bekannten Mündungskontrollen, welche ohnehin nur die Dichtfläche überprüfen könne, weit überlegen ist.

Obwohl die Signaldarstellung in Fig. 2 nur für die Verhältnisse bei einer Flasche mit Gewindeverschluß gelten, läßt sich das erfindugnsgemäße Verfahren auch bei Flaschen mit Kronkorkenverschluß anwenden, da dort ähliche örtliche Intensitätsmodulationen auftreten wie in Fig. 2 dargestellt.

## Patentanspruch

Verfahren zur opto-elektronischen Inspektion von Flaschen, bei dem
—die Flasche auf einem Bahnabschnitt bewegt und gleichzeitig um ihre Längsachse gedreht wird,
—die Flasche während ihrer Rotation um die Längsachse von einer diffus strahlenden Lichtquelle unter einem schiefen Winkel α bezüglich der Flaschenlängsachse beleuchtet wird, und
—das schief durch die Flaschenlängsachse hindurchtretende Licht von einer parallel zur Flaschenlängsachse orientierten Fotodiodenzeile oder einem Fotodiodenarray sequentiell abgetastet und jedes eine Folge übereinander liegender Bildpunkte bzw. Lichtlinie repräsentierende Abtastsignal im Sinne einer Fehlererkennung ausgewertet wird,
dadurch gekennzeichnet, daß die Flasche auf einem Kreisbahnabschnitt bewegt qird und daß zum Überprüfen des Mündungsbereiches der Flasche einschließlich eines Flaschengewindes auf Glasausbrüche die bei der Abtastung hervorgerufene Intensitätsmodulation jedes Abtastsignals dahingehend ausgewertet wird, ob der Abstand zwischen jeweils zwei benachbarten Intensitätsmaxima größer oder kleiner als ein Referenzwert ist, wobei ein Abstandswert oberhalb dieses Referenzwertes einen Glasausbruch identifiziert.

## Revendication

Procédé d'inspection optoélectronique de bouteilles, dans lequel
—la bouteille est déplacée sur une partie de voie et est simultanément tournée autour de son axe longitudinal,
—la bouteille, au cours de sa rotation autour de son axe longitudinal, est éclairée par une source de lumière à rayonnement diffus sous un angle oblique α par rapport à l'axe longitudinal de la bouteille, et
—la lumière pénétrant obliquement par rapport à l'axe longitudinal de bouteille est détectée séquentiellement par une ligne de photodiodes orientée parallèlement à l'axe longitudinal de la bouteille, ou par un réseau de photodiodes, et chaque signal de détection représentant une séquence de points d'image superposé ou ligne lumineuse est évaluée dans le but de déterminer des défauts,
caractérisé en ce que la bouteille est déplacée sur une partie circulaire de voie et que, pour vérifier la présence éventuelle de cassures de verre dans la zone de l'embouchure de la bouteille y compris le filetage de bouteille, la modulation d'intensité de chaque signal de détection, provoquée lors de la détection, est évaluée selon que la distance entre deux maxima voisins d'intensité respectifs est supérieure ou inférieure à une valeur de référence, une valeur de distance supéieure à cette valeur de référence identifiant une cassure du verre.

## Claim

A method for the opto-electronic inspection of bottles, wherein
—the bottle is moved on a portion of a track and is simultaneously rotated about its longitudinal axis,
—during its rotation about the longitudinal axis, the bottle is illuminated by a diffusely radiating light source at an oblique angle α relative to the longitudinal axis of the bottle, and

—the light which has passed obliquely through the longitudinal axis of the bottle is sequentially scanned by a line of photodiodes orientated parallel to the bottle longitudinal axis or by a photodiode array and each scan signal representing a sequence of image points or light lines lying one above another is evaluated in the sense of a fault recognition,

characterized in that the bottle is moved on a portion of a circular track and that, for inspecting the mouth region of the bottle including a bottle thread for glass fractures, the intensity modulation of each scan signal produced in the scanning is evaluated to find whether the difference between each two adjacent intensity maxima is greater than or less than a reference value, a difference above this reference value identifying a glass fracture.

F I G . 1

F I G . 2

Intensität

Ort

Länge einer Diodenzeile